# EUROPEAN PATENT SPECIFICATION

(11) **EP 1 520 527 B1**
(45) Date of publication and mention of the grant of the patent: **02.11.2006**
(21) Application number: 04256018.5
(22) Date of filing: 30.09.2004
(51) Int. Cl.: A61B 17/11

(54) **Anastomosis device**
Vorrichtung für Anastomose
Dispositif d'anastomose

(30) Priority: 30.09.2003 US 675705
(43) Date of publication of application: 06.04.2005
(73) Proprietor: ETHICON ENDO-SURGERY, INC., Cincinnati, Ohio 45242 (US)
(72) Inventor: Ortiz, Mark, Milford Ohio 45150 (US)
(74) Representative: Tunstall, Christopher Stephen

(56) References cited:
- WO-A-03/000142
- US-B1- 6 312 446
- US-B1- 6 461 320
- US-B1- 6 543 456

## Description

### Cross Reference to Related Applications

The present application is related to four co-pending and commonly-owned application filed on even date herewith:
"Anastomosis Wire Ring Device", published as US2005/070934 to Don Tanaka, Mark Ortiz and Darrel Powell;
"Applier For Fastener For Single Lumen Access Anastomosis", published as US2005/07926 to Mark Ortiz;
"Unfolding Anastomosis Ring Device", published as US 2005/07939 to Jean Beaupre; and
"Single Lumen Anastomosis Applier for Fastener",published as US 2005/07921 to Mark Ortiz, Robert McKenna, Bill Kramer, Mike Stokes, and Foster Stulen.

### Field of the Invention

The present invention relates, in general, to devices for surgically modifying organs and vessels. More particularly, it relates to anastomosis devices for joining two organs such as, for example, two separate lengths of small bowel to each other, a section of small bowel to the stomach, or the common bile duct to the duodenum in a procedure called a choledochoduodenostomy. Vascular anastomosis may be performed as well.

### Background of the Invention

The percentage of the world population suffering from morbid obesity is steadily increasing. Severely obese persons are susceptible to increased risk of heart disease, stroke, diabetes, pulmonary disease, and accidents. Because of the effect of morbid obesity to the life of the patient, methods of treating morbid obesity are being researched.

Numerous non-operative therapies for morbid obesity have been tried with virtually no permanent success. Dietary counseling, behavior modification, wiring a patient's jaws shut, and pharmacological methods have all been tried, and though temporarily effective, failed to correct the condition. Further, introducing an object in the stomach, such as an esophago-gastric balloon, to fill the stomach have also been used to treat the condition; however, such approaches tend to cause irritation to the stomach and are not effective long-term.

Surgical treatments of morbid obesity have been increasingly used with greater success. These approaches may be generalized as those that reduce the effective size of the stomach, limiting the amount of food intake, and those that create malabsorption of the food that it is eaten. For instance, some patients benefit from adjustable gastric bands (AGB) that are advantageously laparoscopically placed about the stomach to form a stoma of a desired size that allows food to fill an upper portion of the stomach, causing a feeling of satiety. To allow adjustment of the size of the stoma after implantation, a fluid conduit communicates between an inwardly presented fluid bladder of the AGB to a fluid injection port subcutaneously placed in front of the patient's sternum. A syringe needle may then inject or withdraw fluid as desired to adjust the AGB.

Although an effective approach to obesity for some, other patients may find the lifestyle changes undesirable, necessitated by the restricted amount of food intake. In addition, the medical condition of the patient may suggest the need for a more permanent solution. To that end, surgical approaches have been used to alter the portions of the stomach and/or small intestine available for digesting food. Creating an anastomosis, or the surgical formation of a passage between two normally distinct vessels, is a critical step of many surgical procedures. This is particularly true of gastric bypass procedures in which two portions of small intestine are joined together and another portion of small intestine is joined to the stomach of the patient. This is also true of surgery to alleviate blockage in the common bile duct by draining bile from the duct to the small intestine during surgery for pancreatic cancer.

With particular reference to gastric bypass procedures, current methods of performing a laparoscopic anastomoses for a gastric bypass include stapling, suturing, and placing biofragmentable rings, each having significant challenges. For instance, suturing is time consuming, as well as being technique and dexterity dependent. Stapling requires placement of an anvil, which is a large device that cannot be introduced through a trocar port. Having to introduce the port through a laparotomy presents an increased incidence of wound site infection associated with intralumenal content being dragged to the laparotomy entry site.

As an example of the latter approach, in U.S. Pat. No. 6,543,456 a method for gastric bypass surgery includes the insertion of proximal and distal anastomosis members (e.g., anvils) transorally with grasping forceps. The stomach and the small intestine are transected endoscopically by a surgical severing and stapling instrument to create a gastric pouch, a drainage loop, and a Roux limb. An endoscopically inserted circular stapler attaches to the distal anastomosis member to join the drainage loop to a distal portion of the intestine, and the circular stapler attaches to the proximal anastomosis member to join the Roux limb to the gastric pouch. Thereafter, the anastomosis members are removed to create an orifice between joined portions of the stomach and intestine. This method reduces the number of laparoscopic ports, avoids a laparoscopic insertion of an anastomosis instrument (e.g., circular stapler) into an enlarged surgical port, and eliminates the need for an enterotomy and an enterotomy closure.

For many anastomoses, surgeons use circular staplers, linear staplers, or manual sutures. However, to reduce incision size and to make the surgical process less technically demanding and time consuming, an anastomotic device that deforms to hold tissue portions together when the device is ejected from a constraining enclosure has been described. Such an approach is described in U.S. Pat. Appl. Publ. No. US 2003/0032967 and PCT application WO 03/000142 both to Adrian Park et al, describes such a device. Therein, gastrointestinal or enteric (including biliary) anastomosis is achieved by insertion of a sheath that perforates the walls of two tissue passages, such as the stomach and small intestine. A three-dimensional woven tube of wire of having a thermal shape memory effect (SME) ("generally-known nitinol ring device") is presented by a cannula of the sheath on both sides of the openings. Deployment of the woven tube causes the outer loops or ends of the tube to fold or loop back to hold the luminal interface of the anastomosis site in apposition. Thereby, the need for a mechanical compression component in a delivery system is reduced or avoided, reducing the size and complexity of the delivery device.

The anastomotic device disclosed in WO 03/000142 is constrained by a retractable sheath to an advantageous small-diameter tubular shape. A surgeon applies the anastomotic device by maneuvering the sheath through the tissue portions requiring anastomosis and retracting the sheath. Retracting the sheath removes the constraint on the device, allowing the device to assume a roughly hourglass shape. The larger ends of the hourglass shape hold the two tissue portions together in an effective anastomosis.

The constrained anastomotic device, which may be made of a shape memory material such as nitinol, exerts a force against the inner diameter of the sheath and tends to warp towards its roughly hourglass-shaped deployed position. When the sheath is retracted proximally, the forces generated by the device in transition from a tubular shape to an hourglass shape urge the anastomotic device distally. This device movement makes surgical control harder to achieve when placing the device through the otomies of two tissue portions requiring anastomosis.

While the generally-known nitinol ring device is a significant advancement in the treatment of morbid obesity, it is believed that further improvements would be desirable. For instance, weaving the wire strands and fastening together the ends and heat treating the woven tubes into an SME device is expensive. In addition, it may tend to be difficult to maintain two lumens that are to be anastomatized in extremely close contact in order for the generally-known nitinol ring device to successfully attach to both sides. Having to insert one or more grasping tools along with the anastomosis ring applier tends to mitigate the advantages of a single lumen anastomosis by requiring multiple access ports. Moreover, even if the lumens are proximately position, the generally-known nitinol ring device tends to actuate slowly, if at all, by being limited to SME actuation.

The anastomosis device disclosed in Document US 6.461.320, which corresponds to the preamble of the independent claim 1, discloses a proximal ring, a center ring, a distal ring and a plurality of arms.

Consequently, there is a general need for an device for single lumen access anastomosis that can be used in existing trocar ports (e.g., 12 mm size) and that reliably and effectively creates an anastomotic attachment between lumens, eliminating the need for surgical stapling and suturing to form an anastomosis.

### Brief Summary of the Invention

The invention overcomes the above-noted and other deficiencies of the prior art by providing an anastomosis device according to claim 1, which comprises: a ring device for a single lumen access anastomosis being suitable and sufficient to perform lumen control and apposition as well as enterotomy control. An applier which does not form part of the invention, that may be inserted through a trocar and applied without any additional parts such as an anvil. The applier holds the ring device that has distal and proximal arm segments that the applier individually actuates to enhance control. For instance, the distal arm segments may be expanded in a distal lumen, which is then drawn back into closer contact with the proximal lumen before actuating the proximal arm segment. Alternatively, the proximal arm segments may be expanded first and the first lumen positioned relative to the second lumen. Thereby, positioning the two lumens to be anastomotized is simplified.

In one aspect of the invention, an anastomosis device has an unactuated shape of a cylinder with a proximal ring at one end and a distal ring at the other. A plurality of proximal arms are attached to the proximal ring and a plurality of distal arms are attached to the distal ring. Inwardly directed ends of the distal arms are coupled to inwardly directed ends of the proximal arms at a center portion such that the arms will outwardly actuate when the rings are drawn closer together during actuation. A latching mechanism locks the rings in this actuated shape of a rivet. Thereby, a single lumen procedure is capable where an applier introduces the anastomosis device across the tissue walls and can actuate the device by moving the rings, which automatically latch, allowing the applier to be removed. Over time, the device is passed from the digestive system leaving a patent anastomosis.

These and other objects and advantages of the present invention shall be made apparent from the accompanying drawings and the description thereof.

### Brief Description of the Figures

The accompanying drawings, which are incorporated in and constitute a part of this specification, illustrate embodiments of the invention, and, together with the general description of the invention given above, and the detailed description of the embodiments given below, serve to explain the principles of the present invention.

FIGURE 1 is perspective view of an single lumen access deployable ring for intralumenal anastomosis installed upon an applier being inserted laparoscopically to an anastomosis target site on each of two portions of a patient's small intestine.

FIGURE 2 is the applier of FIG. 1 after actuation of the single lumen access deployable ring to appose the two portions of small intestine.

FIGURE 3 is a detail view of the unactuated single lumen access deployable ring and distal tip and catches of the applier.

FIGURE 4 is a perspective detail view of a partially-actuated ring device and the catches and distal tip of the applier of FIG. 2 .

FIGURE 5 is a side elevation detail view of the partially-actuated ring device and distal portion of the applier of FIG. 2 cutaway along the longitudinal axis.

FIGURE 6 is a perspective detail view of a fully actuated ring device and distal portion of the applier of FIG. 2.

FIGURE 7 is a side elevation view of the fully actuated and deployed ring device of FIG. 6.

### Detailed Description of the Invention

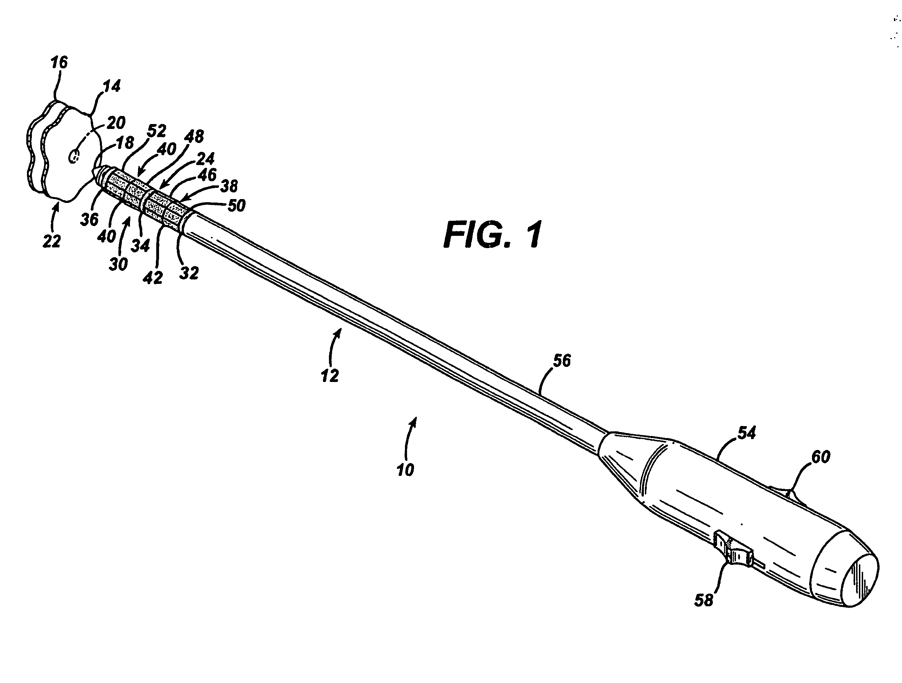

Turning to the Drawings, wherein like numerals denote like components throughout the several views, FIG. 1 depicts an applier 10 which does not form part of the invention, having an elongate implement portion 12 dimensionally sized for insertion through a cannula of a trocar or laparoscopic port to tissues walls 14, 16 to anastomose two lumens. A distal introducer tip 18 of the applier 10 pierces through an opening 20 at an anastomosis site 22 to position an actuating portion 24 that holds a ring device 30 for single lumen anastomosis.

The ring device 30 has three primary rings, depicted as a proximal ring 32, a center ring 34, and a distal ring 36, that are cylindrically aligned with one another. The proximal ring 32 is longitudinally attached to the center ring 34 by proximal arms 38, which in turn is longitudinally attached to the distal ring 36 by distal arms 40. Each proximal and distal arm 38, 40 is bisected respectively by a hinged joint 42, 44 defining an inner arm segment 46, 48 also hingedly attaching to the center ring 34 and an outer arm segment 50, 52 also hingedly attached to the respective proximal or distal ring 32, 36. In its unactuated state as depicted in FIG. 1, the device 30 is cylindrical. The relative lengths of the inner arm segments 46, 48 to outer arm segments 50, 52 may be selected to provide a desired angular contact to tissue walls 14, 16. In the illustrative version, the relationship resembles a cantilevered contact with the inner arm segments 46, 48 actuating to an approximately parallel relationship to the tissue walls 14, 16.

A handle portion 54 is proximally connected to a shaft 56 of the implement portion 12. The shaft 56 may be rigid or flexible, with the latter being desirable for intralumenal insertion, such as through the esophagus. The handle includes controls for longitudinally positioning the rings 32-36 of the ring device 30. In the illustrative version, this includes a center ring slide control 58 and a distal ring slide control 60. Although a manually positioned and actuated applier 10 is depicted for clarity, it should be appreciated that a remotely positioned and actuated applier may be used consistent with aspects of the invention, for instance to allow placement in a more controlled manner, to avoid disturbing an imaging modality, or for other reasons. The handle 54 may further include controls for a distal tip illumination capability so that actuation of the distal arms 40 in the distal lumen may be proximally viewed from an endoscope. It will be appreciated that the terms "proximal" and "distal" are used herein with reference to a clinician gripping the handle portion 54 of the applier 10.

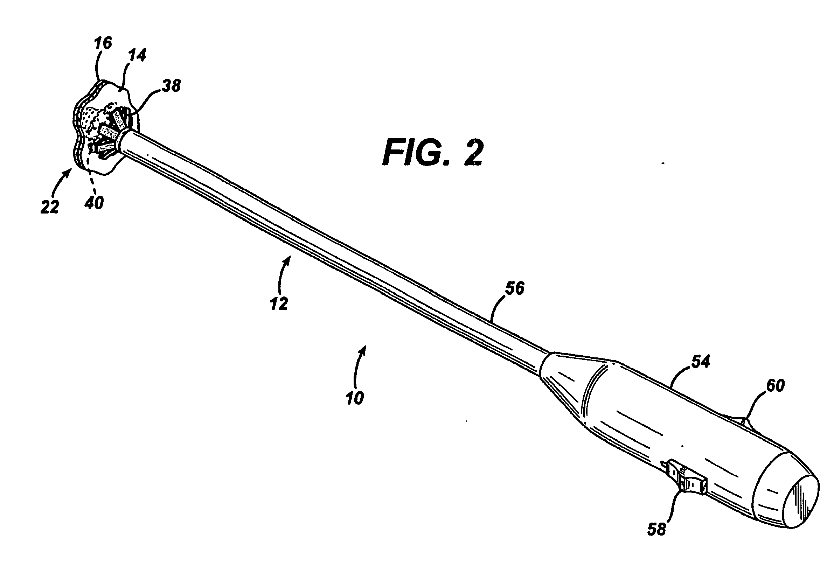

In FIG. 2, two slide controls 58, 62 have been withdrawn proximally, bringing both the center and distal rings 34, 36 into locking proximity of the proximal ring 32, which is held in place by resting against the shaft 56. In response thereto, the proximal and distal arms 38,40 hinge outwardly from the longitudinal axis of the device 30, creating a hollow rivet or hourglass shape for apposing tissue walls 14, 16. The center ring 34 sits at a tissue junction between lumens and the distal and proximal rings 32, 36 come to rest in respective lumens. By latching rings 32-36 one to another when actuated, the device 30 is held in the actuated position with bent arms 38, 40 apposing tissue. The proximal arms 38 may be staggered, as depicted, from distal arms 40 to create a tortuous path for the compressed tissue. Alternatively, the arms 38, 40 may be aligned to directly mate to each other.

It should be appreciated that in the illustrative version, the proximal ring 32 is stationary with respect to the applier 10. In some applications, a third control may be incorporated so that each of the three rings may be positioned independently from the rest, further enhancing the ability to actuate either the distal or the proximal arms 40, 38. As another alternative, the center ring 34 may be stationary with respect to the applier 10, with controls effective to move the proximal and distal rings 32, 36 inwardly to the center ring 34.

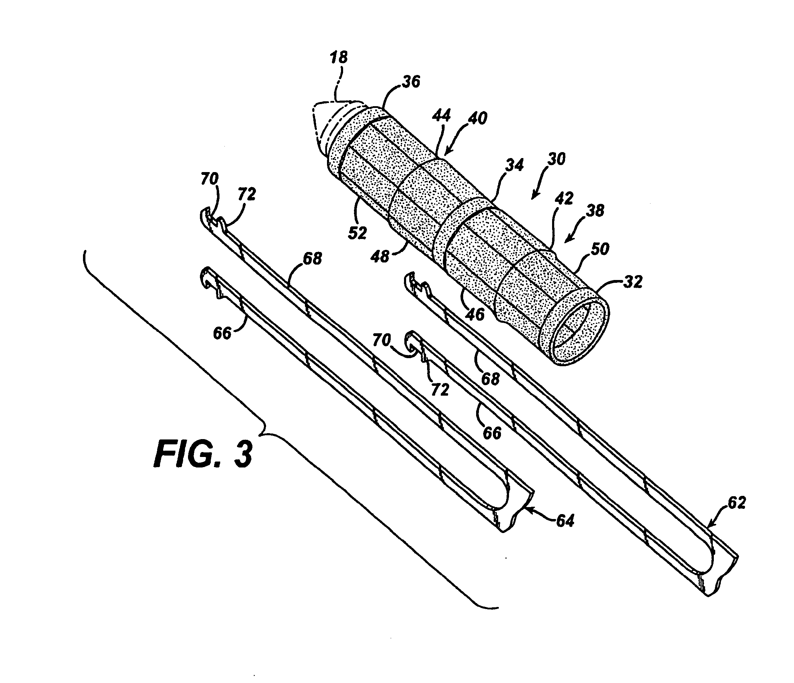

In FIG. 3, the unactuated ring device 30 is shown with the distal introducer tip 18 of the applier 10. The ring device 10 may be comprised of a single piece of molded or stamped material. For instance, the ring device 10 may be advantageously formed from a stamped piece of sheet metal that is wound around a mandrel and tack welded, fused, adhered, etc., into a cylindrical shape. Snap rings may be used at each longitudinal end and the midpoint as well to maintain the shape. Cuts define the arms 38, 40 and creases define the hinged portions. Similar manufacturing economies may be achieved by molding the ring device 30 from a polymeric material. Furthermore, the device 10 may be formed entirely or partially of a biofragmentable or absorbable material to assist in the eventual passing of the device 10, leaving a patent anastomosis. The ring device 10 may advantageously include radiopaque markers in the arms to allow diagnostic imaging to confirm placement of the device 10 and/or to confirm passing. It should be appreciated that the afore-described methods of manufacture are believed to yield economical and therapeutic advantages; however, other techniques for fabrication and assembly may be employed.

Also depicted in FIG. 3, a center ring actuating member 62 and a distal ring actuating member 64 are shown that move within the shaft 56 in response to the center and distal ring slide controls 58, 60. In the illustrative version, each actuating member 62, 64 is formed from a rigid polymer or sheet metal to have two parallel elongate prongs 66, 68 springedly outwardly biased or urged outwardly by other portions of the applier 10 to present distally and laterally presented catches 70 to the inner surface of their respective rings for engagement. Proximal to each catch 70 is a releasing ramp 72 that causes the catch 70 to move inwardly as the releasing ramp 72 contacts the next more proximal ring at or near full actuation. Thus, the ring device 30 is disengaged from the actuating portion 24 of the applier 10 and may be deployed.

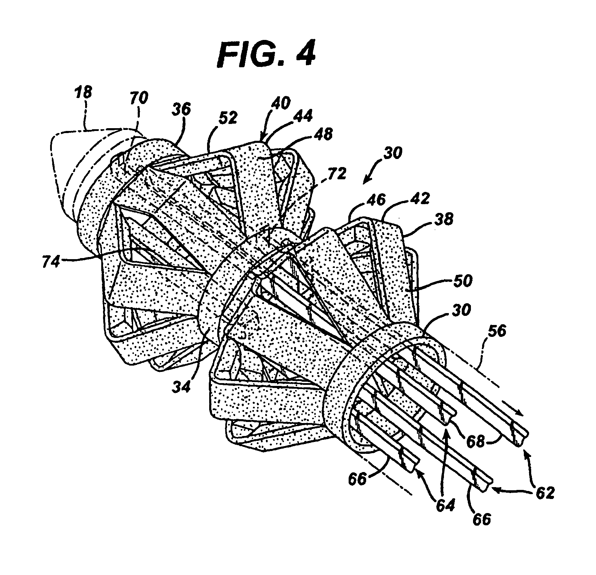

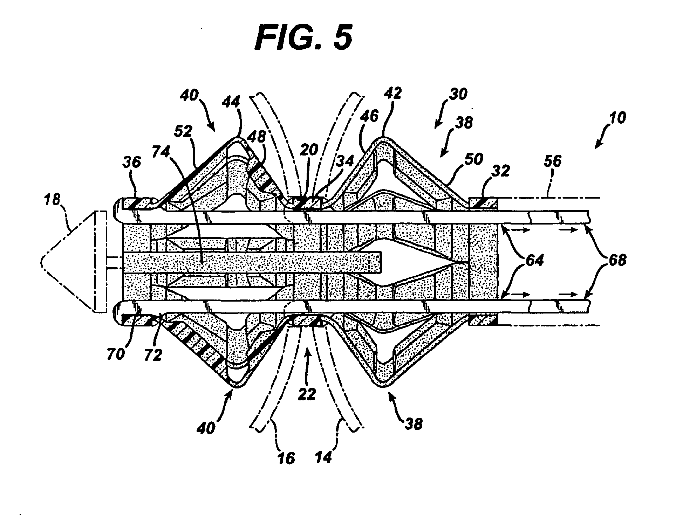

In FIGS. 4-5, the actuating members 62, 64 are depicted as having moved proximally to an intermediate locking position. The shaft 56 (shown in phantom) has restrained the proximal ring 32 while center ring actuating member 62 has drawn back the center ring 34 such that the proximal arms 38 have partially actuated. Similarly, the distal ring actuating member 64 has drawn back the distal ring 36 such that the relative distance between the distal and center 36, 34 is sufficient to also partially actuate the distal arms 40. A locking mechanism, depicted as proximally directed locking hook 74, is connected to the distal ring 36 and is depicted as transitioning past the center ring 34 at this intermediate actuating position. It may be desired in some applications for there to be sufficient interference or latching at intermediate points during actuation for the ring device 30 to remain in a partially actuated position.

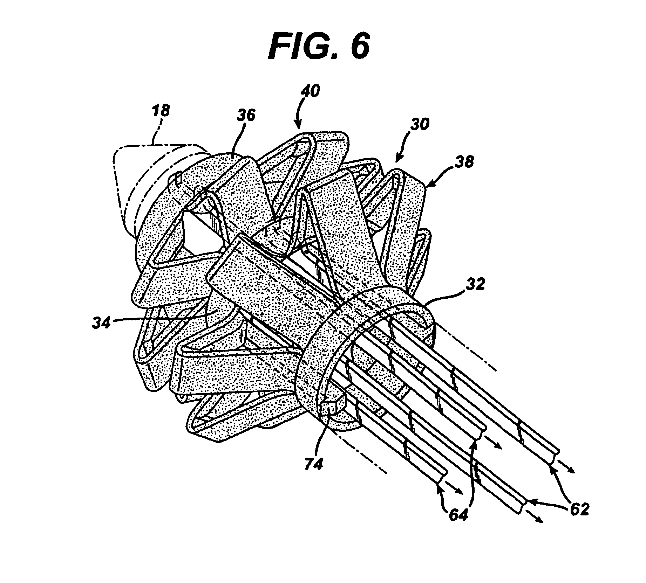

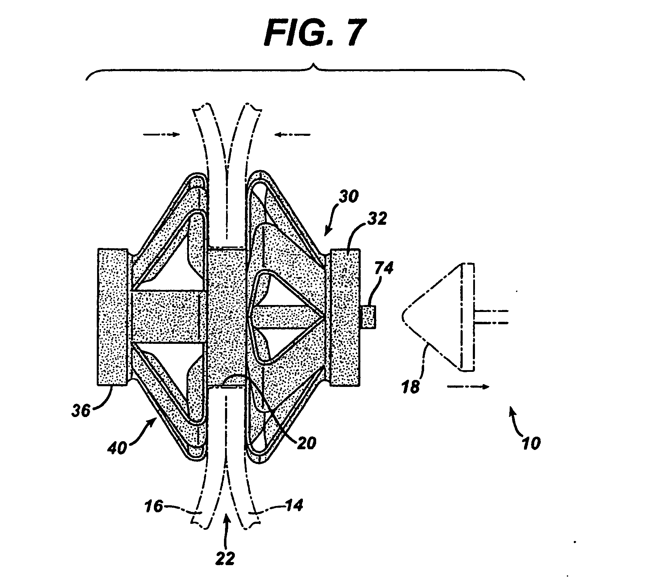

In FIGS. 6-7, the ring device 30 has fully actuated. In FIG. 6, the actuating members 62, 64 have caused the locking hook 74 to lock the distal ring 36 to the proximal ring 32. It should be appreciated that this simple latching mechanism is illustrative and for clarity. A distally presented hook from the proximal ring 32 for instance may intermediately latch to the center ring 34 when the proximal arms 38 are partially actuated and latch to the distal ring 36 when the proximal arms 38 are fully actuated. In FIG. 7, the applier 10 has been withdrawn from the ring device 30. An advantage of having the locking hook exposed in the proximal lumen is convenient access for confirming latching and for reversing the closing of the device 30 in instances where a leak is detected after actuation (e.g., from the opening 20 out between the tissue walls 14, 16).

In use, a ring device 30 is received upon an actuating portion 24 of an implement portion 12 of an applier 10. Specifically, the proximal ring 32 of the device 30 rests against the shaft 56, a center ring actuating member 62 engages the center ring 34 of the device 30, and a distal ring actuating member 64 engages the distal ring 36 of the device 30. A clinician manipulates the handle 54 to insert the implement portion 12 through the cannula of a trocar, laparoscopic port, or through a lumen such as the esophagus to the anastomosis site 22. The tissue walls 14, 16 are proximately placed and the introducer tip 18 of the implement portion 12 passes through the opening 20 formed in these walls 14, 16. The introducer tip may include a piercing shape and/or electromagnetically or thermally enhanced cutting features to assist in forming the opening 20. Once the distal arms 40 of the device 30 are in the distal lumen, the distal ring slide control 60 may be proximally moved to actuate the distal arms into a partially actuated ring shape, latching the locking hook 74 to the center ring 34. The distal tissue wall 16 thus held may be drawn back proximally if necessary such that the proximal arms 38 reside within the first lumen. Drawing back the center ring slide control 58 thus partially actuates the proximal arms 38. If the positioning is correct, the slide controls 58, 60 may be fully slid, latching the locking hook 74 to the proximal ring and causing the proximal and distal arms 38, 40 to be fully actuated and disengaging the catches 70 that hold the applier 10 to the ring device 30. Then, the distal tip 18 of the applier is withdrawn from the ring device 30 leaving it deployed to form the anastomotic attachment. Over time, the tissue walls 14, 16 permanently heal together and the ring device 30 may be passed out of the digestive tract, especially if biofragmentable.

While the present invention has been illustrated by description of several embodiments and while the illustrative embodiments have been described in considerable detail, it is not the intention of the applicant to restrict or in any way limit the scope of the appended claims to such detail. Additional advantages and modifications may readily appear to those skilled in the art.

For example, aspects of the invention have application to surgical procedures not forming part of the invention performed endoscopically and laparoscopically, as well as an open procedure. Use herein of one of these or similar terms should not be construed to limit the present invention for use in only one category of surgical procedure.

For another example, although bariatric procedures for bypassing portions of a gastrointestinal tract are depicted, it should be appreciated that other surgical procedures not forming part of the invention may benefit by an anastomotic ring device having aspects described herein, such as for the bile duct and vascular bypasses.

As an additional example, instead of a center ring 34, the proximal arms 38 may attach to the distal arms 40 in an accordion-like fashion with the proximal ring 32 locking to the distal ring 36. Thus, the center portion of the device 30 at the tissue junction is capable of dilating, thereby further stabilizing the lumens to be anastomosed and preventing tissue slippage. This dilation may be effected either by the proximal and distal rings 32, 36 forcing a center portion to dilate with a wedging action or by making the inner arm segments 46-48 shorter than the outer arm segments 50-52.

As yet a further example, the rings 32, 34, 36 present an intemally projecting contour that may be engaged by the catches 70 of the applier. Other engagements may be incorporated, such as a frangible adhesion between actuating members and one or more rings. In addition, a distal introducer tip may act as an anvil that may be withdrawn proximally to longitudinally compress the device, with features that may be radially withdrawn to thereafter allow the distal introducer tip to be removed from the ring device for deployment.

As yet another example, pads on the inner arm segments may be included to control the pressure profile on the tissue. Comers may be softened or smoothed to avoid any adverse effects of a traumatic contact to tissue.

## Claims

1. An anastomosis device (30), comprising:
a proximal ring (32);
a distal ring (36);
a plurality of proximal arms (38) each attached to the proximal ring (32) at one end and having a distally directed other end;
a plurality of distal arms (40) attached to the distal ring (36) at one end and having a proximally directed other end;
a center portion coupling the proximal end of each distal arm (40) to the distal end of each proximal arm (38); **characterized by**:
a latching mechanism operably configured to lock at a reduced longitudinal spacing two selected from a group consisting of the proximal ring (32), the distal ring (36), and the center portion;
whereby the anastomosis device (30) forms a cylindrical shape when unactuated, and
the proximal and distal arms (38, 40) each outwardly extend when actuated to form a rivet shape, when both the center and distal rings (34, 36) are brought into locking proximity of the proximal ring (32).

2. The anastomosis device of claim 1, wherein the center portion comprises a center ring (34) aligned and interposed between the proximal and distal rings.

3. The anastomosis device of claim 2, wherein the proximal arms (38) are radially aligned with the distal arms 40.

4. The anastomosis device of claim 2, wherein the proximal arms (38) are radially staggered with the distal arm (40) to form a tortuous path of apposed tissue

5. The anastomosis device of claim 1, further comprising radiopaque target material.

6. The anastomosis device of claim 1, wherein the device is formed from polymer material.

7. The anastomosis device of claim 6, wherein the device is formed from biofragmentable material.

8. The anastomosis device of claim 1, wherein the device is formed from sheet material, cylindrically formed onto a mandrel, and opposing longitudinal edges attaches one to another.

9. The anastomosis device of claim 1, wherein the latching mechanism comprises at least one interiorly disposed hook (74).

10. The anastomosis device of claim 1, wherein the latching mechanism comprises an interference fit formed between rings.

## Patentansprüche

1. Vorrichtung (30) für Anastomose, umfassend:
einen proximalen Ring (32),
einen distalen Ring (36),
eine Anzahl von proximalen Armen (38), die jeweils an dem proximalen Ring (32) an einem Ende angebracht sind und ein distal gerichtetes weiteres Ende aufweisen,
eine Anzahl von distalen Armen (40), die an dem distalen Ring (36) an einem Ende angebracht sind und ein proximal gerichtetes weiteres Ende aufweisen,
einen mittigen Abschnitt, der das proximale Ende jedes distalen Arms (40) mit dem distalen Ende jedes proximalen Arms (38) koppelt, **gekennzeichnet durch**
einen Verriegelungsmechanismus, der funktionsmäßig konfiguriert ist, um in einem reduzierten longitudinalen Abstand zwei zu verriegeln, die aus einer Gruppe ausgewählt sind, die aus dem proximalen Ring (32), dem distalen Ring (36) und dem mittigen Abschnitt besteht,
wodurch die Vorrichtung (30) für Anastomose eine zylindrische Gestalt bei Nichtbetätigung bildet und bei Betätigung die proximalen und distalen Arme (38, 40) sich jeweils nach außen erstrecken, um die Gestalt einer Niete zu bilden, wenn sowohl die mittigen als auch distalen Ringe (34, 36) in Verriegelungsnähe des proximalen Ringes (32) gebracht werden.

2. Vorrichtung für Anastomose nach Anspruch 1, **dadurch gekennzeichnet, daß** der mittige Abschnitt einen mittigen Ring (34) umfaßt, der in einer Linie und zwischen den proximalen und distalen Ringen angeordnet ist.

3. Vorrichtung für Anastomose nach Anspruch 2, **dadurch gekennzeichnet, daß** die proximalen Arme (38) radial auf die distalen Arme (40) ausgerichtet sind.

4. Vorrichtung für Anastomose nach Anspruch 2, **dadurch gekennzeichnet, daß** die proximalen Arme (38) zu den distalen Armen (40) radial versetzt sind, um einen gewundenen Weg für anliegendes Gewebe zu bilden.

5. Vorrichtung für Anastomose nach Anspruch 1, **dadurch gekennzeichnet, daß** sie ferner ein strahlenundurchlässiges Zielmaterial umfaßt.

6. Vorrichtung für Anastomose nach Anspruch 1, **dadurch gekennzeichnet, daß** die Vorrichtung aus Polymermaterial ausgebildet ist.

7. Vorrichtung für Anastomose nach Anspruch 6, **dadurch gekennzeichnet, daß** die Vorrichtung aus biologisch abbaubarem Material ausgebildet ist.

8. Vorrichtung für Anastomose nach Anspruch 1, **dadurch gekennzeichnet, daß** die Vorrichtung aus bahnförmigem Material ausgebildet ist, das auf einem Dorn zylindrisch ausgebildet ist, und gegenüberliegende longitudinale Kanten aneinander anliegen.

9. Vorrichtung für Anastomose nach Anspruch 1, **dadurch gekennzeichnet, daß** der Verriegelungsmechanismus mindestens einen innen angeordneten Haken (74) aufweist.

10. Vorrichtung für Anastomose nach Anspruch 1, **dadurch gekennzeichnet, daß** der Verriegelungsmechanismus eine zwischen Ringen ausgebildete Preßpassung aufweist.

## Revendications

1. Dispositif d'anastomose (30), comprenant :
un anneau proximal (32) ;
un anneau distal (36) ;
une pluralité de bras proximaux (38), chacun étant fixé à l'anneau proximal (32), à une extrémité et ayant une autre extrémité dirigée de façon distale ;
une pluralité de bras distaux (40) fixés à l'anneau distal (36) à une extrémité et ayant une autre extrémité dirigée de façon proximale ;
une partie centrale couplant l'extrémité proximale de chaque bras distal (40) à l'extrémité distale de chaque bras proximal (38), **caractérisé par** :
un mécanisme de verrouillage configuré de façon opérationnelle pour bloquer à un espacement réduit longitudinalement deux éléments choisis dans un groupe comprenant l'anneau proximal (32), l'anneau distal (36) et la partie centrale ;
moyennant quoi le dispositif d'anastomose (30) prend une forme cylindrique lorsqu'il n'est pas actionné, et les bras proximal et distal (38, 40) s'étendent chacun vers l'extérieur lorsqu'ils sont actionnés pour prendre une forme de rivet, lorsque les deux anneaux central et distal (34, 36) sont amenés dans la proximité de blocage de l'anneau proximal (32).

2. Dispositif d'anastomose selon la revendication 1, dans lequel la partie centrale comprend un anneau central (34) aligné et intercalé entre les anneaux proximal et distal.

3. Dispositif d'anastomose selon la revendication 2, dans lequel les bras proximaux (38) sont alignés radialement sur les bras distaux 40.

4. Dispositif d'anastomose selon la revendication 2, dans lequel les bras proximaux (38) sont radialement étagés avec les bras distaux (40) pour former un trajet tortueux de tissu apposé.

5. Dispositif d'anastomose selon la revendication 1, comprenant en outre un matériau cible radioopaque.

6. Dispositif d'anastomose selon la revendication 1, dans lequel le dispositif est formé à partir d'un matériau polymère.

7. Dispositif d'anastomose selon la revendication 6, dans lequel le dispositif est formé à partir d'un matériau biofragmentable.

8. Dispositif d'anastomose selon la revendication 1, dans lequel le dispositif est formé à partir d'un matériau de feuille, formé cylindriquement sur un mandrin, et les bords longitudinaux opposés se fixent l'un à l'autre.

9. Dispositif d'anastomose selon la revendication 1, dans lequel le mécanisme de verrouillage comprend au moins un crochet disposé à l'intérieur (74).

10. Dispositif d'anastomose selon la revendication 1, dans lequel le mécanisme de verrouillage comprend un ajustement avec serrage formé entre les anneaux.
